# EUROPEAN PATENT APPLICATION

(11) **EP 4 113 533 A2**
(43) Date of publication of application: **04.01.2023**
(21) Application number: 22180906.4
(22) Date of filing: 24.06.2022
(51) Int. Cl.: G16H 40/40, G16H 20/17, A61B 5/1495, A61B 5/145, A61M 5/142, A61M 5/172

(54) **TRANSITIONS TO REPLACEMENT ON-BODY MEDICAL DEVICES FROM EXPIRING ON-BODY MEDICAL DEVICES THAT AVOID DOWNTIME**

(30) Priority: 30.06.2021 US 202163216824 P
(71) Applicant: Insulet Corporation, Acton MA 01720 (US)
(72) Inventor: ZADE, Ashutosh, San Diego (US); ZHENG, Yibin, Hartland (US); LEE, Joon Bok, Acton (US); O'CONNOR, Jason, Acton (US)
(74) Representative: Peterreins Schley

(57) **Abstract**

The switchover between an expiring on-body medicament delivery device and a replacement on-body medicament is made to eliminate or significantly decrease the time where an on-body medicament delivery device is operational to deliver medicament to a user. The replacement on-body medicament device is attached to the user and prepped for operation while the expiring on-body medicament delivery device is still operational. The switchover between on-body sensors also may be improved. Methods for calibrating a replacement on-body sensor while the expiring on-body sensor is still operative are provided. The calibrating may be performed quickly so that there is no gap in operation between expiration of the expiring on-body sensor and full operation of the replacement on-body sensor.

## Description

### BACKGROUND

Certain on-body medical devices expire after a period of use. These on-body medical devices must be detached from their users upon expiration and replaced with replacement on-body medical devices. Examples of such on-body medical devices include medicament delivery devices, such as insulin pumps, and glucose monitors, such as continuous glucose monitors (CGMs).

The switchover from an expiring on-body medical device to a replacement medical device can have some problems. For example, with insulin pumps, there may be a period of time where an expiring insulin pump has been deactivated and a replacement pump is attached to the user but not yet operative. This situation arises in part because the insulin pump has a long warm-up period after being activated before the insulin pump becomes operative. The situation also arises in part because it may take a non-negligible amount of time (e.g., approximately 20 minutes) to establish wireless communications between the replacement insulin pump and a glucose monitor. Since the replacement insulin pump relies on readings from the glucose monitor to determine what insulin doses to deliver, the replacement insulin pump is not operative until the wireless communications are established and a current blood glucose concentration reading is received from the glucose monitor. As a result, a user may not have an operative insulin pump for the period between the expiring insulin pump being deactivated and the replacement insulin pump being operative.

Another example of a problem concerns on-body glucose monitors. When an expiring on-body glucose monitor is replaced by a replacement on-body glucose monitor, there is a period before the replacement on-body glucose monitor can provide accurate readings. This period is known as the "stabilization period." The stabilization period typically is in the range of 60 to 120 minutes. During the stabilization period there are no accurate blood glucose concentration readings, and thus, there is no way of knowing if the user is at risk of imminent hypoglycemia during the stabilization period.

Other problems arise when an on-body medical device expires, and the user is unable to immediately replace the expired on-body medical device with a replacement device.

### SUMMARY

In accordance with an inventive aspect, a method that is performed by a processor includes preparing a second on-body medicament delivery device with the processor to replace a first on-body medicament delivery device that has an expiration while the first on-body medicament delivery device is positioned on a user and is operational to deliver a medicament to the user. The preparing includes placing the second on-body medicament delivery device in a standby mode with the processor where the second on-body medicament delivery device is not operational to deliver the medicament to the user but is powered up. The preparing by the processor also includes receiving and storing data and communication information from the first on-body medicament delivery device at the second on-body medicament delivery device and priming a pump of the second on-body medicament delivery device so that the pump is ready to deliver the medicament to the user. The second on-body medicament delivery device is activated by the processor to become operational to deliver the medicament to the user based on the expiration of the first on-body medicament delivery device.

The medicament may be one of insulin, glucagon or a glucagon-like peptide (GLP)-1 receptor agonist, for example. The medicament may include at least one of: a chemotherapeutic agent, a pain relief agent, a blood thinner agent, a hormonal agent, a pharmaceutical agent or a therapeutic agent. The received and stored data may include blood glucose history for the user and delivery history of the agent for the user. The method may include notifying the user that the second on-body medicament delivery device is ready for activating after the preparing is complete. The second on-body medicament delivery device may have a controller, and the controller may perform the placing of the second on-body medicament delivery device in a standby mode. The activating may be responsive to a received communication. The preparing may further include initiating communication set up between a sensor and the second on-body medicament delivery device at a predetermined period of time before expiration of the first on-body medicament delivery device. The sensor may be a glucose monitor for monitoring blood glucose levels of the user.

In accordance with another inventive aspect, a method is provided for preparing a second on-body sensor for use with a user while a first on-body sensor is in use. Per the method, the second on-body sensor that is attached to the user is placed in a warming-up mode while the first on-body sensor is attached to the user and is sensing an analyte or other physical characteristic. The warming-up mode prepares the second on-body sensor to be activated to sense the analyte or the other physical characteristic while attached to the user. The second on-body sensor is calibrated to accurately sense the analyte or the other physical characteristic while still in the warming up mode.

The method may include activating the second on-body sensor to begin sensing the analyte or other physical characteristic while attached to the user. The on-body sensors may include a continuous blood glucose monitor, a lactate sensor, a heart rate sensor or other type of sensor. The analyte or other sensed physical characteristic may be one of lactate, a hormone, a drug concentration, heart rate, blood pressure, galvanic skin response, respiration rate, concentration of a substance in blood of the user, or presence of a substance in the blood of the user. The method may include receiving at the second on-body sensor a reading of the analyte or of the other physical characteristic from the first on-body sensor to be used in calibrating the second on-body sensor.

The calibrating may include determining a delta between the received reading of the analyte or of the other physical characteristic from the first on-body sensor and a current reading of the analyte or of the other physical characteristic read by the second on-body sensor and using the delta in the calibrating of the second on-body sensor. The first on-body sensor and the second on-body sensor may be positioned a known or pre-instructed distance apart on the user by the user, and the calibrating may be based in part upon a time it takes for a tracer solution introduced into the user to be sensed by the respective first and second on-body sensors. The calibrating may be based on the user ingesting a calibration liquid or a predetermined quantity of commercially available liquid drink and monitoring a change in blood glucose concentration with the second on-body sensor. The calibrating may include communicating with a controller device to obtain information to calibrate the second on-body sensor.

In accordance with an additional inventive aspect, an on-body medical device includes a storage for storing processor-executable instructions and a processor for executing the instructions. The processor executes the instructions to prepare a second on-body medical device to replace the first on-body delivery device while a first on-body medical device that has an expiration is positioned on a user and is operational. The prepping includes placing the second on-body medical device in a standby mode where the second on-body medical device is not fully operational but is powered up and establishing wireless communications with at least another device. The processor also executes the instructions to transition from the standby mode to an operational mode in which the on-body medical device is fully operational. The second device may refrain from being fully operational during preparation to allow the first medical device to run to expiration and to enhance the life of the second medical device, such as a sensor on the second medical device, for example.

### BRIEF DESCRIPTION OF THE DRAWINGS

Figure 1 depicts an illustrative medicament delivery system suitable for exemplary embodiments.
Figure 2 depicts a flowchart of illustrative steps that may be performed in exemplary embodiments to switchover from an expiring on-body medicament delivery device to a replacement on-body medicament delivery device.
Figure 3 depicts a user with an attached expiring on-body medicament delivery device and an attached replacement on-body medicament delivery device.
Figure 4 depicts a flowchart of illustrative steps that may be performed in exemplary embodiments to prepare for a switchover.
Figure 5 depicts a flowchart of illustrative steps that may be performed in exemplary embodiments to prepare the replacement on-body medicament delivery device.
Figure 6 depicts a flowchart of illustrative steps that may be performed in exemplary embodiments to initialize wireless communications with sensors.
Figure 7A depicts a flowchart of illustrative steps that may be performed in exemplary embodiments in accordance with a first option to inform the user of expiration and activate the replacement on-body medicament delivery device.
Figure 7B depicts a flowchart of illustrative steps that may be performed in exemplary embodiments in accordance with a second option to inform the user of expiration and activate the replacement on-body medicament delivery device.
Figure 7C depicts a flowchart of illustrative steps that may be performed in exemplary embodiments in accordance with a third option to inform the user of expiration and activate the replacement on-body medicament delivery device.
Figure 8 depicts a flowchart of illustrative steps that may be performed in exemplary embodiments in accordance with a first option to perform a switchover from an expiring on-body sensor to a replacement on-body sensor.
Figure 9 depicts a diagram of a user with an expiring on-body sensor and a replacement on-body sensor attached to different sides of his/her lower back.
Figure 10 depicts a flowchart of illustrative steps that may be performed in exemplary embodiments in accordance with a second option to perform a switchover from an expiring on-body sensor to a replacement on-body sensor.
Figure 11 depicts a flowchart of illustrative steps that may be performed in exemplary embodiments in accordance with a third option to perform a switchover from an expiring on-body sensor to a replacement on-body sensor.
Figure 12 depicts a flowchart of illustrative steps that may be performed in exemplary embodiments in accordance with a fourth option to perform a switchover from an expiring on-body sensor to a replacement on-body sensor.

### DETAILED DESCRIPTION

The exemplary embodiments may improve the transition from expiring on-body medical devices to replacement on-body medical devices. For example, the exemplary embodiments may improve this transition for on-body medicament delivery devices, like insulin pumps. The exemplary embodiments may activate a replacement on-body medicament delivery device while an expiring on-body medicament delivery device is still attached to a user and still operative. This allows the replacement medicament device to warm up while the expiring on-body medicament delivery device is still operative. The replacement on-body medicament delivery device may receive communication information needed for communication with other devices and other data needed for operation from the expiring on-body medicament delivery device. The initialization of wireless communications with other devices may begin before expiration of the expiring on-body medicament delivery device.

As a result, the replacement on-body medicament device may be ready to begin operation at the time of expiration and/or time of removal of the expiring on-body medicament device from the user. The exemplary embodiments may reduce the down time of the replacement on-body medicament delivery device. The exemplary embodiments also may eliminate the need to replace the expiring pumps at inopportune times; rather, with the exemplary embodiments, the replacement on-body medicament device may be automatically activated without user input at the appropriate time (e.g., upon expiration of the first or expired on-body medicament device) and the expiring on-body medicament delivery device may be removed when it is convenient.

The exemplary embodiments additionally enable communications with other devices to smoothly transition to the replacement on-body medicament delivery device. Communication handoffs from the expiring on-body medicament delivery device to the replacement on-body medicament delivery device for communications with devices, such as glucose monitors, may be initiated a set period of time before the replacement occurs. This provides sufficient time so that the steps needed to realize the handoff are completed in time for the transition.

The exemplary embodiments may also facilitate a smooth transition between an expiring on-body sensor, such as a glucose monitor, and a replacement on-body sensor. In the exemplary embodiments, the replacement sensor is inserted into the user and allowed to warm up. To shorten the period of time it takes for the on-body sensor to warm up, the exemplary embodiments may provide techniques for quickly calibrating the replacement sensor so that the warm-up period may be shortened substantially. In one exemplary embodiment, the expiring on-body sensor communicates with the replacement on-body sensor to calibrate the replacement on-body sensor. In another exemplary embodiment, a management device calibrates the replacement on-body sensor. In still another exemplary embodiment, the calibration of the replacement on-body sensor entails introducing an agent into the user that has a known effect on sensed analyte levels and calibrating the replacement on-body sensor from analyte levels sensed by the replacement on-body sensor after introduction of the agent.

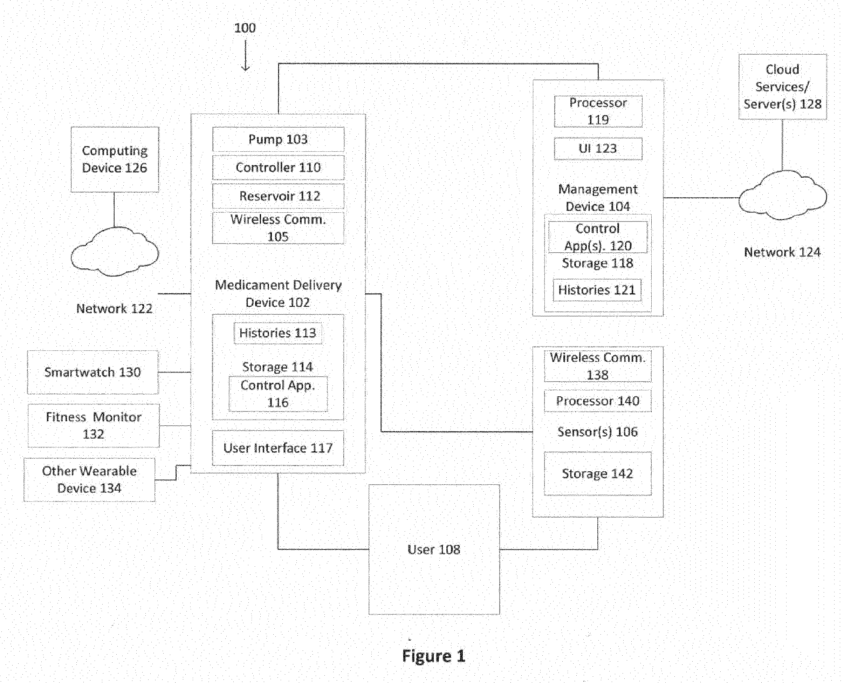

Figure 1 depicts an illustrative medicament delivery system 100 that is suitable for delivering a medicament to a user 108 in accordance with exemplary embodiments. The medicament delivery system 100 includes a medicament delivery device 102. The medicament delivery device 102 may be a wearable device that is worn on the body of the user 108 or carried by the user and which has an infusion site. Alternatively, the medicament delivery device 102 may be an on-body device that is directly coupled to a user (e.g., directly attached to a body part and/or skin of the user 108 via an adhesive or the like) with tubing and a cannula or needle connecting the medicament delivery device 102 to an infusion site where the medicament is injected. In a preferred embodiment, a surface of housing(s) of the medicament delivery device 102 may include an adhesive to facilitate attachment to the user 108.

The medicament delivery device 102 may include a controller 110. The controller 110 may be implemented in hardware, software, or any combination thereof. The controller 110 may, for example, include a microprocessor, a logic circuit, a field programmable gate array (FPGA), an application specific integrated circuit (ASIC) or a microcontroller coupled to a memory. The controller 110 may maintain a date and time as well as other functions (e.g., calculations or the like). The controller 110 may be operable to execute a control application 116 stored in the storage 114 that enables the controller 110 to direct operation of the delivery device 102. The control application 116 may control delivery of the medicament to the user 108 per a control approach like that described herein. The storage 114 may hold histories 113 for a user, such as a history of basal deliveries, a history of bolus deliveries, a history of blood glucose concentrations and/or other histories, such as a meal event history, exercise event history hypoglycemia and/or hyperglycemia events history and/or the like. In addition, the controller 110 may be operable to receive data or information, including communication information that enables wireless communication with other devices. The storage 114 may include both primary memory and secondary memory. The storage 114 may include random access memory (RAM), read only memory (ROM), optical storage, magnetic storage, removable storage media, solid state storage or the like.

The medicament delivery device 102 may include one or more housings for housing its various components. The medicament delivery device 102 may include a pump 103 for pumping medicament from the reservoir 112. A fluid path to the user 108 may be provided, and the medicament delivery device 102 may expel the medicament from the reservoir 112 using the pump 103 to deliver the medicament to the user 108 via the fluid path. The fluid path may, for example, include tubing coupling the delivery device 102 to the user 108 (e.g., tubing coupling a cannula to the reservoir 112), and may include tubing to a separate infusion site.

There may be one or more communications links with one or more devices physically separated from the medicament delivery device 102 including, for example, a management device 104 of the user and/or a caregiver of the user and/or sensor(s) 106. The communication links may include any wired or wireless communication links operating according to any known communications protocol or standard, such as Bluetooth^{®}, Wi-Fi, a near-field communication standard, a cellular standard, or any other wireless protocol. A wireless communication integrated circuit (IC) 105 may be provided for participating in wireless communications according to wireless protocols, such as those enumerated above. The wireless communication IC 105 may include a wireless transceiver. The medicament delivery device 102 may also include a user interface 117, such as an integrated display device for displaying information to the user 108 and in some embodiments, receiving information from the user 108. The user interface 117 may include a touchscreen and/or one or more input devices, such as buttons, knobs, or a keyboard.

The medicament delivery device 102 may interface with a network 122. The network 122 may include a local area network (LAN), a wide area network (WAN) or a combination therein. A computing device 126 may be interfaced with the network, and the computing device may communicate with the medicament delivery device 102.

The delivery system 100 may include sensor(s) 106 for sensing the levels of one or more analytes. The sensor(s) 106 may be coupled to the user 108 by, for example, adhesive or the like and may provide information or data on one or more medical conditions and/or physical attributes of the user 108. The sensor(s) 106 may, in some exemplary embodiments provide periodic blood glucose concentration measurements and may be a continuous glucose monitor (CGM), or another type of device or sensor that provides blood glucose measurements. The sensor(s) 106 may be physically separate from the medicament delivery device 102 or may be integrated components thereof. A wireless communication IC 138 may be provided in the sensor(s) to facilitate wireless communications with other devices. The sensor(s) 106 also may include a processor 140 (such as a CPU, GPU, FPGA or ASIC) for executing computer programming instructions. A storage 142 may be provided for storing data and/or computer programming instructions. The storage 142 may be realized as multiple or any one of different type of memory or storage devices, such as described herein above. The sensor(s) 106 may provide the controller 110 with data indicative of measured or detected blood glucose levels of the user 108. The sensor(s) 106 alternatively may measure things such as blood pressure, heart rate, blood alcohol, galvanic skin response, temperature, amount of an analyte in blood or in interstitial fluid. The information or data provided by the sensor(s) 106 may be used to adjust delivery operations of the medicament delivery device 102.

The medicament delivery system 100 may also include a management device 104. In some embodiments, no management device 104 is needed as medicament delivery device 102 may manage itself. The management device 104 may be a special purpose device, such as a dedicated personal diabetes manager (PDM) device. The management device 104 may be a programmed general-purpose device, such as any portable electronic device including, for example, a dedicated controller, such as processor, a micro-controller, or the like. The management device 104 may be used to program or adjust operation of the medicament delivery device 102 and/or the sensor(s) 106. The management device 104 may be any portable electronic device including, for example, a dedicated device, a smartphone, a smartwatch or a tablet. In the depicted example, the management device 104 may include a processor 119 and a storage 118. The processor 119 may execute processes to manage a user's blood glucose levels and to control the delivery of the medicament to the user 108. The processor 119 may also be operable to execute programming code stored in the storage 118. For example, the storage 118 may be operable to store one or more control applications 120 for execution by the processor 119. The one or more control applications 120 may be responsible for controlling the medicament delivery device 102, such as by controlling the AID delivery of insulin to the user 108. The storage 118 may store the one or more control applications 120, histories 121 like those described above for the medicament delivery device 102, and other data and/or programs.

The management device 104 may include a user interface (UI) 123 for communicating with the user 108. The user interface 123 may include a display, such as a touchscreen, for displaying information. The touchscreen may also be used to receive input when it is a touch screen. The user interface 123 may also include input elements, such as a keyboard, button, knobs, or the like.

The management device 104 may interface with a network 124, such as a LAN or WAN or combination of such networks. The management device 104 may communicate over network 124 with one or more servers or cloud services 128.

Other devices, like smartwatch 130, fitness monitor 132 and wearable device 134 may be part of the delivery system 100. These devices may communicate with the medicament delivery device 102 to receive information and/or issue commands to the delivery device 102. These devices 130, 132 and 134 may execute computer programming instructions to perform some of the control functions otherwise performed by controller 110 or processor 119. These devices 130, 132 and 134 may include displays for displaying information. The display may show a user interface for providing input by the user, such as to request a change or pause in dosage or to request, initiate, or confirm delivery of a bolus of a, medicament or for displaying output, such as a change in dosage (e.g., of a basal delivery amount) as determined by controller 110 or management device 104. These devices 130, 132 and 134 may also have wireless communication connections with the sensor 106 to directly receive analyte measurement data.

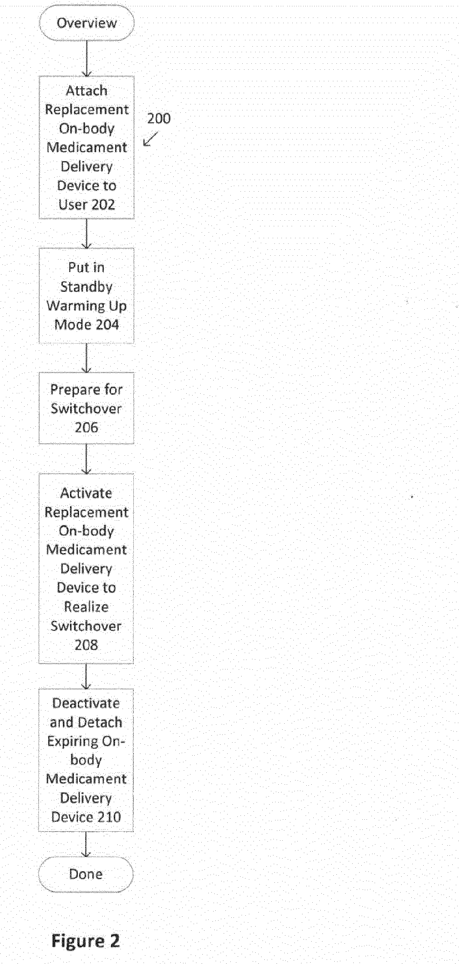

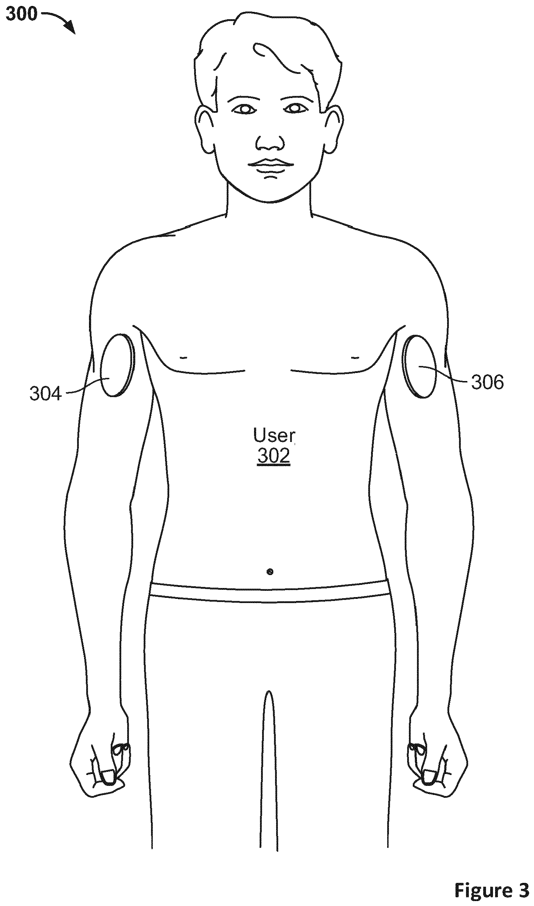

The on-body medicament delivery device 102 expires after a period of time, such as after three days, and must be replaced at least in part with a replacement on-body medicament delivery device. The exemplary embodiments provide an improved approach to making the switchover from the expiring on-body medicament device to the replacement on-body medicament delivery device. Figure 2 depicts a flowchart 200 of illustrative steps that may be performed by exemplary embodiments to realize the switchover. At 202, the replacement on-body medicament delivery device is attached to the user 108. The replacement on-body medicament delivery device may have an adhesive or other securing feature for securing to the skin of the user 108. Figure 3 shows a depiction 300 of an instance wherein a replacement on-body medicament delivery device 306 has been secured to the back of an arm of a user 302. The needle or cannula provided in the replacement on-body medicament delivery device 306 has not yet been deployed. The expiring on-body medicament delivery device 304 is secured to the user 302 on the other arm. The expiring on-body medicament delivery device 304 is operational and delivering medicament to the user 302. The expiration of the expiring on-body medicament delivery device 304 is approaching.

At 204, the replacement on-body medicament delivery device 306 is put in a standby warming-up mode in which the replacement on-body medicament delivery device 306 is powered up and initializing but not yet operational for delivering medicament. Since the replacement on-body medicament delivery device 306 is in this mode, there is no need to enter the warming-up mode after the expiring on-body medicament delivery device 304 has expired and been removed. This approach eliminates the window of time where neither the expiring on-body medicament device 304 or the replacement on-body medicament delivery device 306 is operational to deliver medicament. During the standby warming up mode at 206, the replacement on-body medicament delivery device 306 takes steps to prepare for the switchover, which will be described below. After these steps have been completed, the replacement on-body medicament delivery device 306 is activated to become operational to realize the switchover at 208. The expiring on-body medicament delivery device is deactivated and detached from the user 302 at 210. The deactivation may be the result of the expiration being reached or by explicit deactivation.

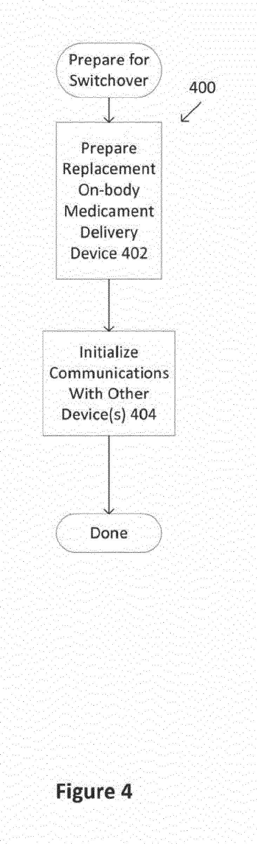

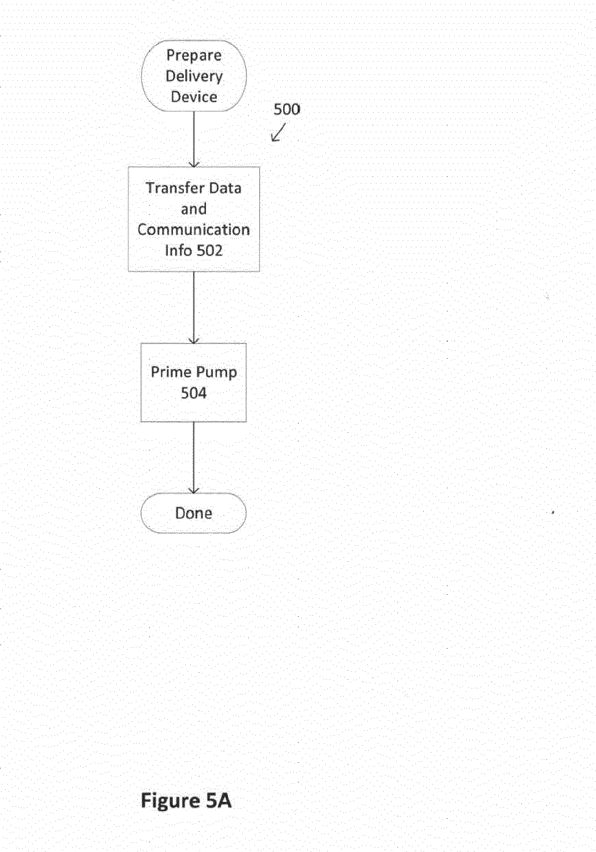

Preparing for the switchover (see 206 in Figure 2) involves operations like those shown in the flowchart 400 of Figure 4. At 402, the replacement on-body medicament delivery device 306 is prepared. Figure 5A depicts a flowchart 500 of illustrative steps that may be performed in exemplary embodiments to prepare the replacement on-body medicament delivery device 306. At 502, data (such as insulin delivery history, blood glucose histories, final state of the expiring on-body medicament device 304 and replacement on-body medicament activation parameters like total daily insulin (TDI) for the user 108, blood glucose concentration target) is transferred to the replacement on-body medicament delivery device 306. Communication information may also be transferred. The communication information includes information needed to wirelessly communicate with other devices, such as device ID's, serial numbers, encryption keys, pairing codes and the like. The transfer may be via the management device 104 and/or may be directly from the expiring on-body medicament delivery device. The replacement on-body medicament delivery device 306 uses the data in its control system. At 504, the pump 103 is primed so that it is ready for use.

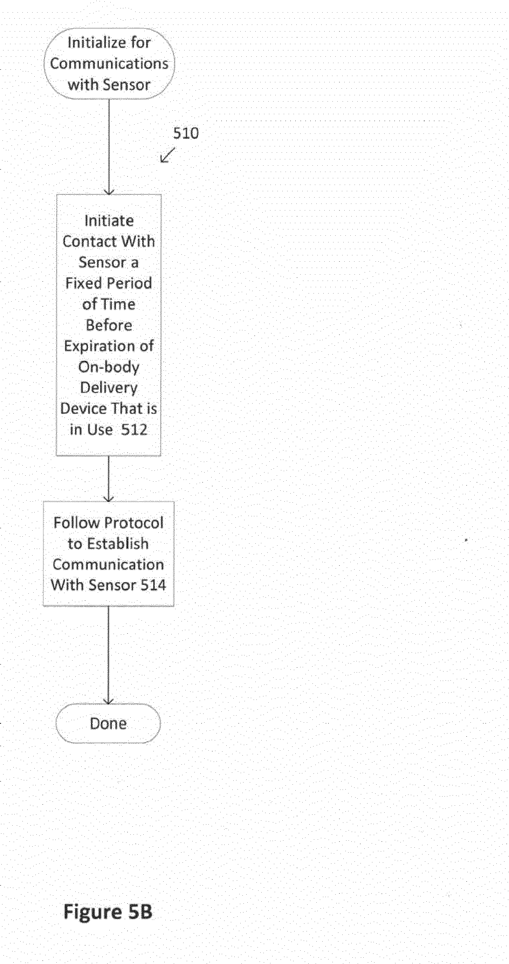

With reference to Figure 4, at 404 wireless communication with other devices including, the sensor(s) 106, such as a blood glucose sensor (e.g., a CGM), the management device 104, the smartwatch 130, the fitness monitor 132, and other wearable device 134, are established. Figure 5B depicts a flowchart 510 of illustrative steps that may be performed by exemplary embodiments to establish wireless communications with these other devices. These steps may be performed particularly for devices that require a substantial time to establish communications and where wireless communications with the devices are needed as soon the replacement on-body medicament delivery device is activated. At 512, wireless contact with a sensor 106 is initiated a fixed period of time before expiration of the expiring on-body medicament delivery device 304 occurs. For example, it may take at least 20 minutes to establish wireless communications with certain CGMs. Thus, the initial communications with such a CGM is initiated 20 minutes before expiration of the expiring on-body medicament delivery device 304; or stated another way, communications with a CGM may be established a period of time before expiration of the on-body medicament delivery device 304, where the period of time is at least as long as the time it takes to establish wireless communication between a new on-body medicament delivery device 306 and the CGM. At 514, the protocol for establishing wireless communication with the sensor 106 is followed. Each variety and brand of sensor may have its own custom protocol for establishing wireless communication. In the exemplary embodiments, the process is initiated before expiration to avoid a gap in time where the replacement on-body medicament delivery device cannot communicate with the sensor 106, like a CGM and hence does not have access to current sensor values, like current blood glucose concentration values for the user 108.

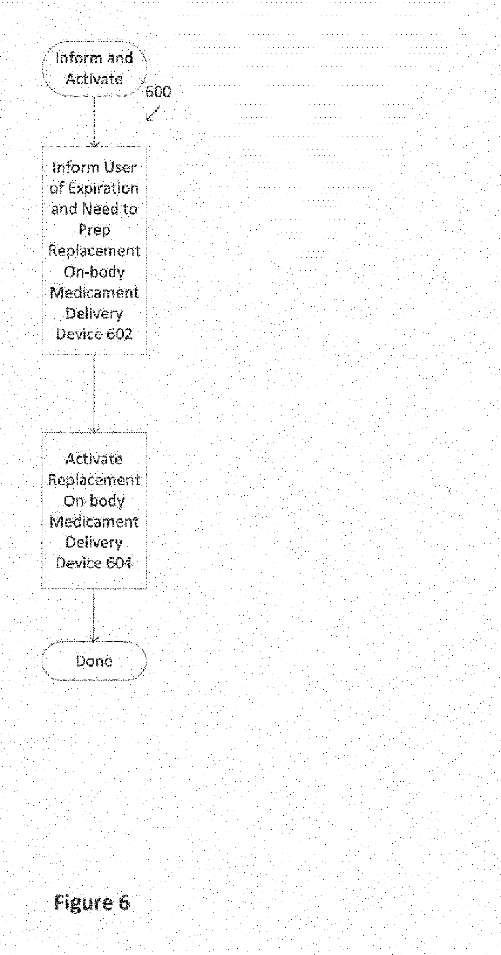

In order to realize the switchover, exemplary embodiments may perform the illustrative steps of flowchart 600 of Figure 6. At 602, the controller 110 may inform the user of the upcoming expiration of the expiring on-body medicament delivery device 304 and the need to prepare the replacement on-body medicament delivery device 306 to be ready for use. The controller may cause messages to be displayed on the user interface. In some exemplary embodiments, the messages may be displayed on the user interface 123 of the management device or even on the smartwatch, the fitness monitor or other wearable device 134. The messages may comprise a time when the on-body medicament delivery device 304 is going to expire, a countdown timer of when the on-body medicament delivery device 304 is going to expire, the time when a new on-body medicament delivery device 306 should be placed on the body, a suggested location of where to place the new on-body medicament delivery device 306, and/or an option to be reminded in the future to place the new on-body medicament delivery device 306 on the body. The UI depicting these messages may show a clock, a timer, and/or may change in color as the time gets closer to when the on-body medicament delivery device 304 is going to expire. At 604, the replacement on-body medicament delivery device 306 then is activated.

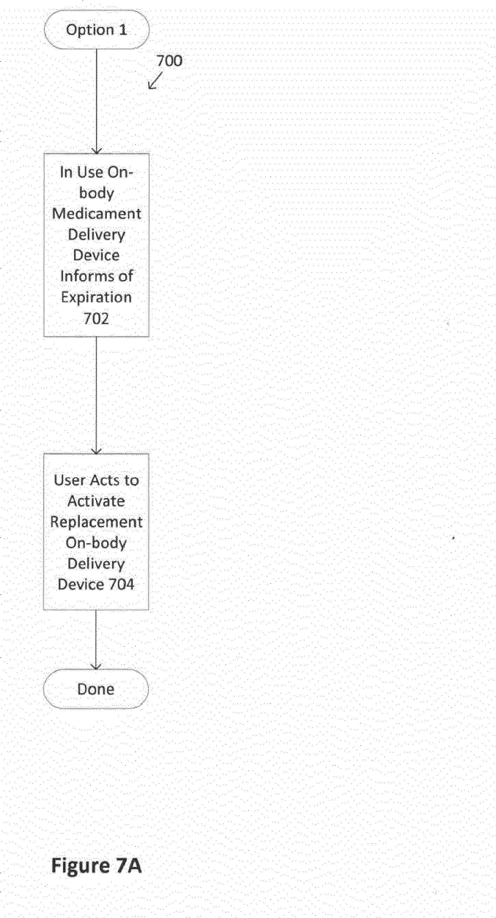

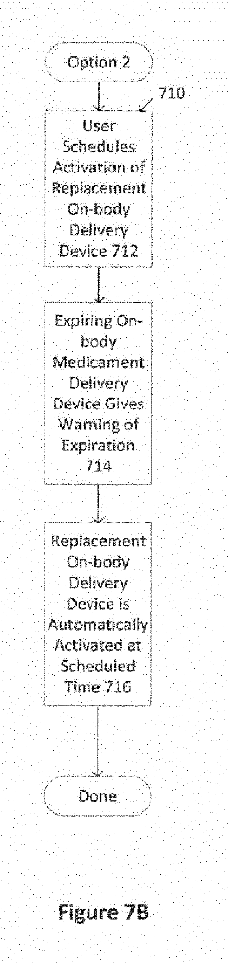

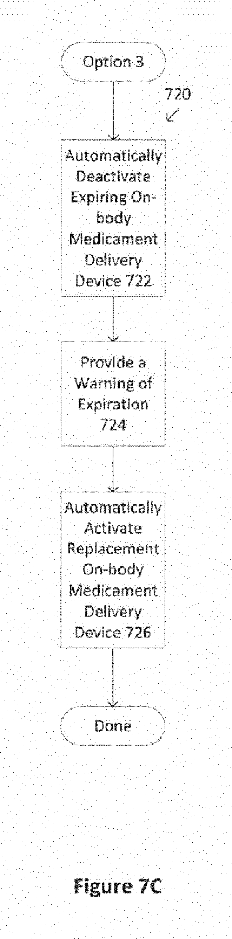

There are several options of how the user 108 may be informed of the need for a switchover (see 602) and the subsequent switchover to the replacement on-body medicament delivery device 306 (see 604). Figures 7A, 7B and 7C show some of the options. These options are meant be illustrative and not exhaustive. Figure 7A shows a flowchart 700 of a first option. At 702, the expiring on-body medicament delivery device 304 informs the user of the upcoming expiration, such as by displaying information on user interface 117. When the replacement on-body medicament delivery device 306 is ready, the user 108 activates the replacement on-body medicament delivery device 306 at 704. The user 108 may press an activation button, issue a command via the management device 104 or otherwise take a step to activate the replacement on-body medicament delivery device 306.

Figure 7B depicts a flowchart 710 of a second option. At 712, the user 108 schedules the activation of the replacement on-body medicament delivery device 306. This may be done by command, such as by using the management device 104. At 714, as the scheduled expiration time approaches, the expiring on-body medicament delivery device 304 gives a warning of the approaching expiration. The warning may be displayed on the user interface 117 of the expiring on-body medicament delivery device 304 or the user interface 123 of the management device 104. Another alternative is to produce an audio output that acts as a warning. The audio output may accompany the textual or graphical output via the user interface 117 or 123. At 716, the replacement on-body delivery device 306 is automatically activated at the scheduled time. This approach provides the user 108 with the convenience of scheduling the switchover at a convenient time. The user 108 avoids undesirable switchover times, such as when at work or school, when sleeping or other inconvenient times.

Figure 7C depicts a flowchart 720 of illustrative steps that may be performed in exemplary embodiments in accordance with a third option. In the third option, at 722, the expiring on-body medicament delivery device 304 is automatically deactivated by the control application 116 or 120. A warning, such as described above relative to 714 is provided at 724. At 726, the replacement on-body medicament delivery device 306 is automatically activated. This third option has the benefit of being fully automatic. This helps to avoid the user 108 forgetting a step or not performing a step properly.

As mentioned above, difficulties with switchovers from expiring on-body medical devices and replacement on-body medical devices are not limited to on-body medicament delivery devices. Difficulties also arise with switchovers between expiring on-body sensors and replacement on-body sensors. The exemplary embodiments may eliminate these difficulties by speeding up the time for the on-body sensors to be ready for use after deployment. For example, conventional sensors or CGMs take 1 or 2 hours after being inserted into the users to be operative and providing proper readings. This is due in part to the time it takes for stabilization so that the CGM may be properly calibrated to yield accurate values.

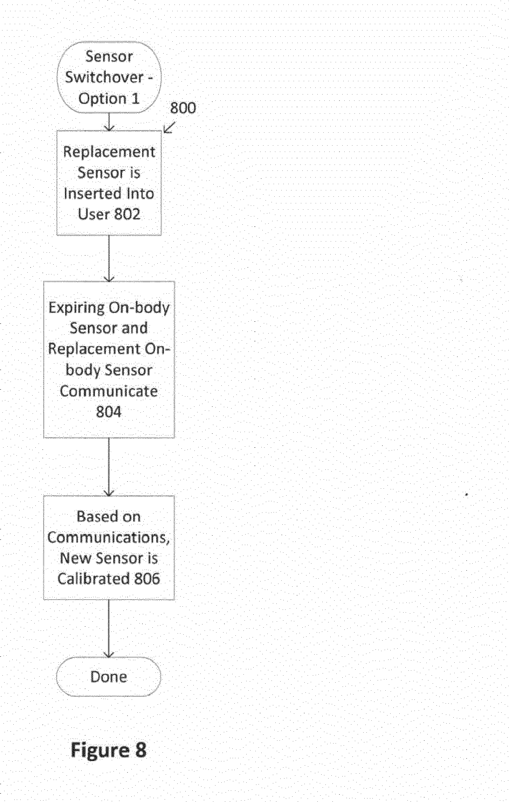

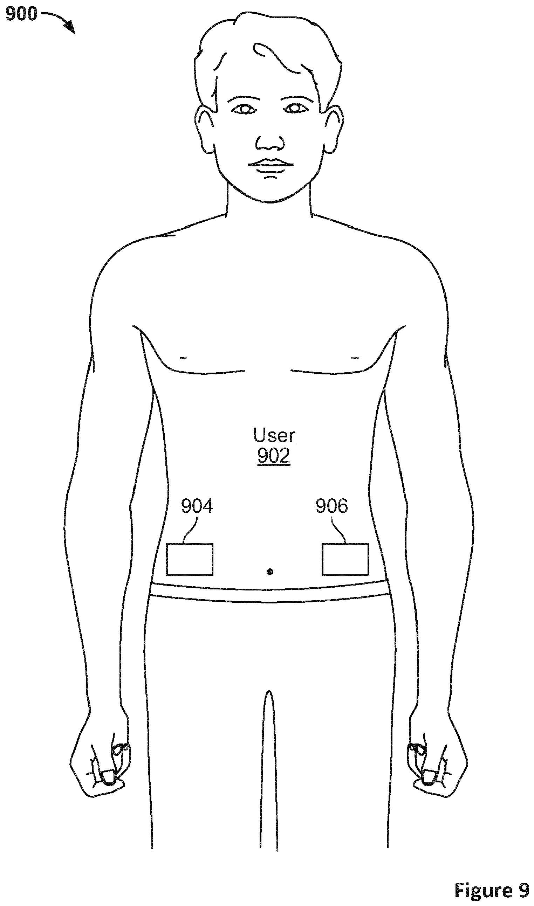

Figure 8 depicts a flowchart 800 of illustrative steps that may be performed in the exemplary embodiments according to a first option. At 802, the replacement on-body sensor is inserted into the user 108. Figure 9 depicts a diagram of a user 902 that has an expiring on-body sensor 904 and a replacement on-body sensor 906 and different sides of his/her back. The expiring on-body sensor 904 remains operative to provide sensor values. The expiring on-body sensor 904 is properly calibrated to provide accurate sensor values. At 804, the expiring on-body sensor 904 and the replacement on-body sensor 906 communicate to share sensor values or other sensor data, such as calibration data or historical data. The expiring on-body sensor 904 may communicate an accurate or calibrated sensor value to the replacement on-body sensor 906. At 806, the replacement on-body sensor 906 using the communicated value may properly calibrate itself and thus be ready to operate. In particular, the offset between the sensor value of the expiring on-body sensor 904 and the replacement on-body sensor 906 is determined and used to calibrate the replacement on-body sensor 906.

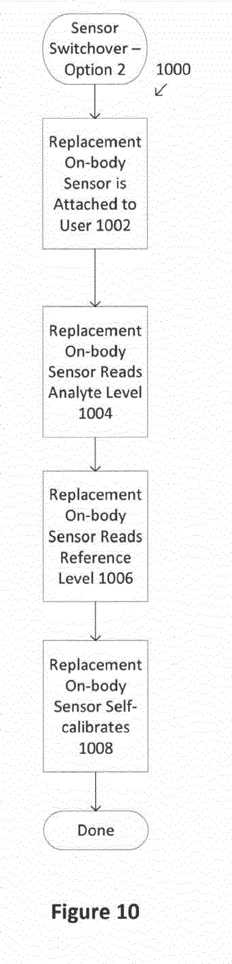

Figure 10 depicts a flowchart of illustrative steps that may be performed by exemplary embodiments to realize the switchover according to a second option. At 1002, the replacement on-body sensor 906 is attached to the user 108. At 1004, the replacement on-body sensor 906 reads an analyte level for the analyte being sensed. At 1006, the replacement on-body sensor 906 receives a reference level. For example, the reference level may be a sensor value of the analyte from a properly calibrated sensor. For a CGM, the user may prick his/her finger to get a drop of blood and process the drop of blood with a blood glucose sensor that is known to be accurate and properly calibrated. The resulting value from that blood glucose sensor may be the reference value. At 1008, the replacement on-body sensor 906 calibrates itself using the reference value.

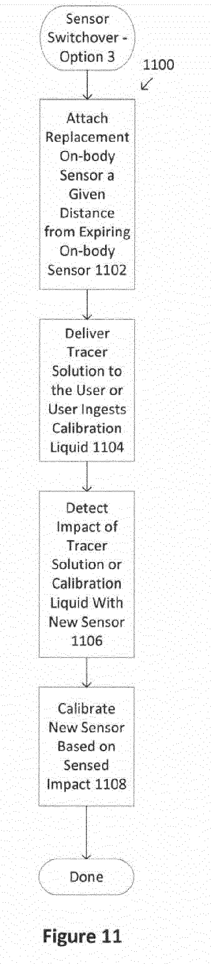

Figure 11 depicts a flowchart 1100 of illustrative steps that may be performed in exemplary embodiments according to a third option for the on-body sensor switchover. This third option relies upon delivery of a tracer solution or ingestion of a calibration liquid to create a disturbance in blood glucose level in order to calibrate the replacement on-body sensor. At 1102, the replacement on-body sensor 906 is attached to the user 108 at a known distance relative to the expiring on-body sensor 904. At 1104, A tracer solution is delivered to the user 108 such as by injection at an injection site on the user or a calibration liquid is delivered to the user 108. The injection site is a known distance from the site of the replacement on-body sensor 906. The tracer solution may be, for example, a sugar solution. The calibration liquid may be, for example, a fast absorbing carbohydrate drink. The quantity of the fast absorbing carbohydrate drink ingested by the user may be gauged by the weight of the user. Thus, the quantity ingested or injected is known, the expected rise in blood glucose concentration is known and the expected time for the rise in blood glucose is known based on the distance between the injection site and the sensor or the expected time for the ingested drink to result in blood glucose in the user. At 1106, the impact of the tracer solution or the calibration liquid on the analyte level sensed by the replacement on-body sensor 906 is detected. The time it takes for the expiring on-body sensor and time it takes for the replacement on-body sensor to detect the rise in blood glucose concentration resulting from the tracer solution. These times may depend on where the on-body sensors are located. For example, an on-body sensor on the lower abdomen may detect a rise in blood glucose concentration prior to an on-body sensor or the arm. At 1108, the detected impact is used to calibrate the replacement on-body sensor 906 relative to the known expected rise in blood glucose concentration. The difference in time of detection by the expiring on-body sensor and the replacement on-body sensor is observed and noted in calibrating the replacement on-body sensor. For example, if there is a five-minute delay in observed changes in the on-body sensors, this delay is noted and used in calibrating the replacement on-body sensor.

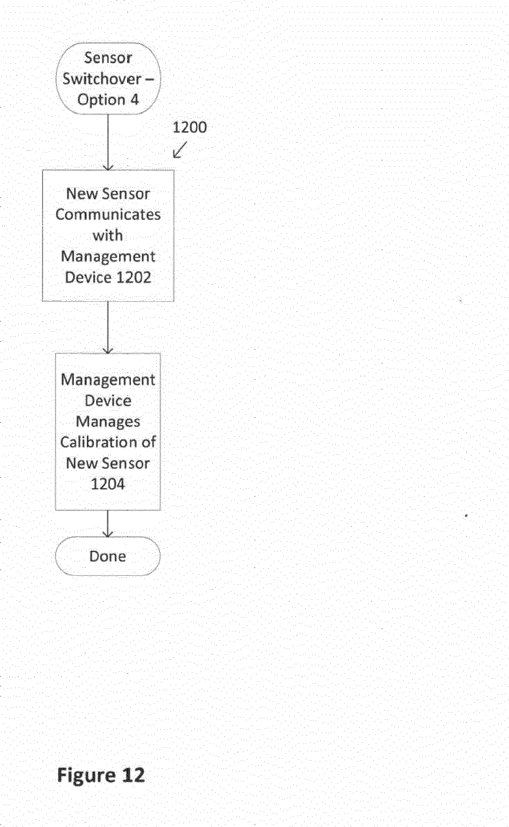

Figure 12 depicts a flowchart 1200 of illustrative steps that may be performed in exemplary embodiments per a fourth option for the on-body sensor switchover. At 1202, the replacement on-body sensor 906 communicates with the management device 104. At 1200, the management device 104 oversees the calibration of the replacement on-body sensor 906. In particular, the management device 104 may manage the replacement on-body sensor and put it into a standby mode in which calibration takes place.

While exemplary embodiments have been described herein, various changes in form and detail may be made relative to the exemplary embodiments without departing from the intended scope of the claims appended hereto.

Although the present invention is defined in the attached claims, it should be understood that the present invention can also (alternatively) be defined in accordance with the following embodiments:
1. A method performed by a processor, comprising:
   preparing a second on-body medicament delivery device to replace a first on-body medicament delivery device that has an expiration with the processor while the first on-body medicament delivery device is positioned on a user and is operational to deliver a medicament to the user, the preparing including with the processor:
   placing the second on-body medicament delivery device in a standby mode in which the second on-body medicament delivery device is not operational to deliver the medicament to the user but is powered up;
   receiving and storing data and communication information from the first on-body medicament delivery device at the second on-body medicament drug delivery device;
   priming a pump of the second on-body medicament delivery device so that the pump is ready to deliver the medicament to the user; and
   based on the expiration of the first on-body medicament delivery device, activating the second on-body medicament delivery device with the processor to become operational to deliver the medicament to the user.
2. The method of embodiment 1, wherein the medicament is one of insulin, glucagon or a glucagonlike peptide (GLP)-1 receptor agonist.
3. The method of one of the preceding embodiments, wherein the medicament includes at least one of a chemotherapeutic agent, a pain relief agent, a blood thinner agent, a hormonal agent, a pharmaceutical agent or a therapeutic agent.
4. The method of one of the preceding embodiments, wherein the received and stored data includes blood glucose history for the user and delivery history of the medicament for the user.
5. The method of one of the preceding embodiments, further comprising notifying the user that the second on-body medicament delivery device is ready for activating after the preparing is complete.
6. The method of one of the preceding embodiments, wherein the second on-body medicament delivery device has a controller and the controller performs the placing of the second on-body medicament delivery device in a standby mode.
7. The method of one of the preceding embodiments, wherein the activating is responsive to a received communication.
8. The method of one of the preceding embodiments, wherein the preparing further comprises initiating communication set up between a sensor and the second on-body medicament delivery device at a predetermined period of time before the expiration of the first medicament on-body delivery device.
9. The method of one of the preceding embodiments, wherein the sensor is a glucose monitor for monitoring blood glucose levels of the user.
10. A method, for preparing a second on-body sensor for use with a user while a first on-body sensor is in use, the method comprising:
   placing the second on-body sensor that is attached to the user in a warming up mode while the first on-body sensor is also attached to the user and is sensing an analyte or other physical characteristic, wherein the warming up mode prepares the second on-body sensor to be activated to sense the analyte or the other physical characteristic while attached to the user; and
   while still in the warming up mode, calibrating the second on-body sensor to accurately sense the analyte or the other physical characteristic.
11. The method of embodiment 10, further comprising activating the second on-body sensor to begin sensing the analyte or the other physical characteristic while attached to the user.
12. The method of one of embodiments 10 to 11, wherein the analyte is blood glucose.
13. The method of one of embodiments 10 to 12, wherein the sensor is a continuous glucose monitor (CGM), a lactate sensor or a heart rate sensor.
14. The method of one of embodiments 10 to 13, wherein the analyte or the other physical characteristic is one of
   lactate, a hormone, a drug concentration, heart rate, blood pressure, galvanic skin response, respiration rate, concentration of a substance in blood of the user, or presence of a substance in the blood of the user.
15. The method of one of embodiments 10 to 14, further comprising receiving at the second on-body sensor a reading of the analyte or the other physical characteristic from the first on-body sensor to be used in the calibrating.
16. The method of one of embodiments 10 to 15, wherein the calibrating comprises:
   determining a delta between the received reading of the analyte or of the other physical characteristic from the first on-body sensor and a current reading of the analyte or of the other physical characteristic read by the second on-body sensor; and
   using the delta in the calibrating of the second on-body sensor.
17. The method of one of embodiments 10 to 16, wherein the first on-body sensor and the second on-body sensor are positioned a known distance apart on the user and wherein the calibrating is based in part upon a time it takes for a tracer solution or disturbance introduced into the user to be sensed by the respective first and second on-body sensors.
18. The method of one of embodiments 10 to 17, wherein the calibrating is based the user ingesting a calibration liquid and monitoring a change in blood glucose concentration with the second on-body sensor.
19. The method of one of embodiments 10 to 18, wherein the calibrating comprises communicating with a controller device to obtain information to calibrate the second on-body sensor.
20. An on-body medical device, comprising:
   a storage for storing processor-executable instructions;
   a processor for executing the instruction to:
      while a first on-body medical device that has an expiration is positioned on a user and is operational, preparing a second on-body medical device to replace the first on-body delivery device by:
      placing the second on-body medical device in a standby mode where the second device is not fully operational but is powered up;
      establishing wireless communications with at least another device; and
      transitioning from the standby mode to an operational mode in which the on-body medical device is fully operational.
21. An on-body medicament delivery device comprising a processor and/or a controller and a storage for storing programming code for executing the method according to one of embodiments 1 to 19.
22. On-body medicament delivery device according to embodiment 21, wherein the storage is designed to store histories for a user, such as a history of basal deliveries, a history of bolus deliveries, a history of blood glucose concentrations and/or other histories, such as a meal event history, exercise event history hypoglycemia and/or hyperglycemia events history and/or the like.

## Claims

1. A method performed by a processor, comprising:
preparing a second on-body medicament delivery device to replace a first on-body medicament delivery device that has an expiration with the processor while the first on-body medicament delivery device is positioned on a user and is operational to deliver a medicament to the user, the preparing including with the processor:
placing the second on-body medicament delivery device in a standby mode in which the second on-body medicament delivery device is not operational to deliver the medicament to the user but is powered up;
receiving and storing data and communication information from the first on-body medicament delivery device at the second on-body medicament drug delivery device;
priming a pump of the second on-body medicament delivery device so that the pump is ready to deliver the medicament to the user; and
based on the expiration of the first on-body medicament delivery device, activating the second on-body medicament delivery device with the processor to become operational to deliver the medicament to the user.

2. The method of claim 1, wherein the medicament is one of insulin, glucagon or a glucagon-like peptide (GLP)-1 receptor agonist.

3. The method of one of the preceding claims, wherein the medicament includes at least one of a chemotherapeutic agent, a pain relief agent, a blood thinner agent, a hormonal agent, a pharmaceutical agent or a therapeutic agent.

4. The method of one of the preceding claims, wherein the received and stored data includes blood glucose history for the user and delivery history of the medicament for the user.

5. The method of one of the preceding claims, further comprising notifying the user that the second on-body medicament delivery device is ready for activating after the preparing is complete.

6. The method of one of the preceding claims, wherein the second on-body medicament delivery device has a controller and the controller performs the placing of the second on-body medicament delivery device in a standby mode.

7. The method of one of the preceding claims, wherein the activating is responsive to a received communication.

8. The method of one of the preceding claims, wherein the preparing further comprises initiating communication set up between a sensor and the second on-body medicament delivery device at a predetermined period of time before the expiration of the first medicament on-body delivery device.

9. The method of one of the preceding claims, wherein the sensor is a glucose monitor for monitoring blood glucose levels of the user.

10. A method, for preparing a second on-body sensor for use with a user while a first on-body sensor is in use, the method comprising:
placing the second on-body sensor that is attached to the user in a warming up mode while the first on-body sensor is also attached to the user and is sensing an analyte or other physical characteristic, wherein the warming up mode prepares the second on-body sensor to be activated to sense the analyte or the other physical characteristic while attached to the user; and
while still in the warming up mode, calibrating the second on-body sensor to accurately sense the analyte or the other physical characteristic.

11. The method of claim 10, further comprising activating the second on-body sensor to begin sensing the analyte or the other physical characteristic while attached to the user.

12. The method of one of claims 10 to 11, wherein the analyte or the other physical characteristic is one of lactate, a hormone, a drug concentration, heart rate, blood pressure, galvanic skin response, respiration rate, concentration of a substance in blood of the user, or presence of a substance in the blood of the user.

13. The method of one of claims 10 to 12, further comprising receiving at the second on-body sensor a reading of the analyte or the other physical characteristic from the first on-body sensor to be used in the calibrating.

14. The method of one of claims 10 to 13, wherein the calibrating comprises:
determining a delta between the received reading of the analyte or of the other physical characteristic from the first on-body sensor and a current reading of the analyte or of the other physical characteristic read by the second on-body sensor; and
using the delta in the calibrating of the second on-body sensor.

15. The method of one of claims 10 to 14, wherein the first on-body sensor and the second on-body sensor are positioned a known distance apart on the user and wherein the calibrating is based in part upon a time it takes for a tracer solution or disturbance introduced into the user to be sensed by the respective first and second on-body sensors.
